(19) **Europäisches Patentamt / European Patent Office / Office européen des brevets**

(11) **EP 4 653 090 A1**

(12) **EUROPEAN PATENT APPLICATION**
published in accordance with Art. 153(4) EPC

(43) Date of publication:
26.11.2025  Bulletin 2025/48

(21) Application number: 24744824.4

(22) Date of filing: 15.01.2024

(51) International Patent Classification (IPC):
$B01J\ 37/00^{(2006.01)}$  $B01J\ 37/08^{(2006.01)}$
$B01J\ 37/04^{(2006.01)}$  $B01J\ 27/199^{(2006.01)}$
$C07C\ 51/25^{(2006.01)}$  $C07C\ 57/04^{(2006.01)}$

(52) Cooperative Patent Classification (CPC):
(C-Sets available)
B01J 27/199; B01J 23/002; B01J 23/8877;
B01J 37/00; B01J 37/0045; B01J 37/02;
B01J 37/04; B01J 37/08; C07C 51/252    (Cont.)

(86) International application number:
PCT/KR2024/000673

(87) International publication number:
WO 2024/155043 (25.07.2024 Gazette 2024/30)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 19.01.2023  KR 20230008209
12.01.2024  KR 20240005265

(71) Applicant: LG Chem, Ltd.
Yeongdeungpo-gu
Seoul 07336 (KR)

(72) Inventors:
• LEE, Saeha
  Daejeon 34122 (KR)
• SEO, Dong Woo
  Daejeon 34122 (KR)
• CHOI, Byung Yul
  Daejeon 34122 (KR)
• PARK, Hyun Woo
  Daejeon 34122 (KR)
• JOO, Hyeonho
  Daejeon 34122 (KR)
• CHOE, Young Hyun
  Daejeon 34122 (KR)
• KIM, Minsu
  Daejeon 34122 (KR)
• LIM, Seulgi
  Daejeon 34122 (KR)
• LIM, Hyunsub
  Daejeon 34122 (KR)
• CHOI, Youngju
  Daejeon 34122 (KR)

(74) Representative: Goddar, Heinz J.
Boehmert & Boehmert
Anwaltspartnerschaft mbB
Pettenkoferstrasse 22
80336 München (DE)

(54) **CATALYST FOR PRODUCING (METH)ACRYLIC ACID, AND METHOD FOR PRODUCING CATALYST FOR PRODUCING (METH)ACRYLIC ACID**

(57)    The present invention provides a catalyst for preparing (meth)acrylic acid and a method for preparing a catalyst for preparing (meth)acrylic acid.

EP 4 653 090 A1

**(Cont. next page)**

(52) Cooperative Patent Classification (CPC): (Cont.)

C-Sets
**C07C 51/252, C07C 57/04**

EP 4 653 090 A1

## Description

[Technical Field]

This application claims priority to and the benefit of Korean Patent Application Nos. 10-2023-0008209 and 10-2024-0005265 filed in the Korean Intellectual Property Office on January 19, 2023 and January 12, 2024, respectively, the entire contents of which are incorporated herein by reference.

The present invention relates to a catalyst for preparing (meth)acrylic acid and a method for preparing a catalyst for preparing (meth)acrylic acid.

[Background Art]

Generally, as the process of preparing (meth)acrylic acid, gas-phase contact oxidization methods of propylene, isobutylene, tertiary butanol, and the like with molecular oxygen in a multitubular fixed bed reactor in which a catalyst layer is present have been used. Since the reaction is an exothermic reaction, various methods such as limiting the thickness of a catalyst layer and using a supported catalyst in which a catalytically active material is supported on a carrier have been applied in order to minimize the increase in temperature during the reaction.

As is known, a method of performing heat treatment is applied to the preparation of the supported catalyst by dispersing a catalytically active material or catalyst precursor in a solvent to obtain a uniform solution or slurry, and then spraying or impregnating the uniform solution or slurry to a carrier or coating the carrier with a powder. Further, to the preparation of an extruded catalyst, a method of dispersing a catalytically active material or catalyst precursor in a solvent to obtain a uniform solution or slurry, then drying the uniform solution or slurry using a drier, pulverizing the dried material using a pulverizer to prepare a powder, adding a molding additive to the powder, and then performing extrusion molding is applied. The extruded catalyst is subjected to a firing process, which is an additional heat treatment, to prepare a final catalyst.

In this case, the firing process is generally performed through a primary firing process and a secondary firing process. In this case, since it takes a long time to perform the firing process, there have been problems in that productivity is decreased and equipment for the firing process becomes complicated.

Therefore, there is a continuing need for research to improve the firing process.

[Citation List] (Patent Document 1) Korean Patent Application Laid-Open No. 10-2014-0138081

[Detailed Description of the Invention]

[Technical Problem]

The present invention has been made in an effort to provide a method for preparing a catalyst for preparing (meth) acrylic acid with an improved firing process, and a catalyst for preparing (meth)acrylic acid prepared using the same.

[Technical Solution]

An exemplary embodiment of the present invention provides a method for preparing a catalyst for preparing (meth)acrylic acid represented by the following Chemical Formula 1, the method including: preparing a first solution including a molybdenum precursor and a vanadium precursor; preparing a second solution including a precursor including an $M^2$ element and a precursor including an $M^4$ element; preparing a catalyst suspension by mixing the first solution and the second solution; preparing a slurry by stirring and reacting the catalyst suspension; drying the slurry; pulverizing the dried slurry, and then adding a molding additive and molding the resulting mixture; and firing the molded catalyst according to the following Equation 1 while supplying a mixed gas including air and nitrogen ($N_2$).

In addition, an exemplary embodiment of the present invention provides a catalyst for preparing (meth)acrylic acid, which is prepared by the method for preparing (meth)acrylic acid, in which the catalyst satisfies the following Chemical Formula 1.

[Chemical Formula 1] $\quad Mo_aV_bP_cM^1_dM^2_eM^3_fM^4_gM^5_hM^6_iO_j$

In Chemical Formula 1, Mo is molybdenum, V is vanadium, P is phosphorus, and O is oxygen,

$M^1$ is one or more elements selected from the group consisting of W, Sb, As, Sn, and Pb,
$M^2$ is one or more elements selected from the group consisting of Fe, Zn, Cr, Mn, and Cu,

$M^3$ is one or more elements selected from the group consisting of Se, Ga, Ti, Ge, Cd, Ta, and Ni,

$M^4$ is one or more elements selected from the group consisting of Al, Zr, Si, and Ce,

$M^5$ is one or more elements selected from the group consisting of Au, Pd, Pt, Ag, Ru, and Rh,

$M^6$ is one or more elements selected from the group consisting of Na, K, Li, Rb, Cs, Ca, Mg, Sr, and Ba,

a, b, c, d, e, f, g, h, i, and j represent the atomic proportion of each element, and

when a is 12, b is 0 to 2, c is 0 to 3, d is 0.001 to 15, e is 0.001 to 20, f is 0 to 20, g is 0 to 10, h is 0 to 1, i is 0.001 to 5, and j is a numerical value determined by the oxidation state of each component,

[Equation 1]

$$0.1 \ \mathrm{Lmin}^{-1}\mathrm{g}^{-1} \leq A/B \leq 5 \ \mathrm{Lmin}^{-1}\mathrm{g}^{-1}$$

in Equation 1,

A means the flow rate ($\mathrm{Lmin}^{-1}$) of the mixed gas, and B means the mass (g) of the molded catalyst.

[Advantageous Effects]

The method for preparing a catalyst for preparing (meth)acrylic acid according to the present invention can simplify and automate the process of preparing a catalyst by performing only one firing process without dividing the firing process into a primary firing process and a secondary firing process, and reduce investment costs by downsizing the equipment according to the preparation process.

Furthermore, since only one firing process is performed, the process of preparing a catalyst can be installed in a smaller space, and since the equipment can be downsized, the risks of the preparation process, such as the risk of leakage of chemicals and the risk of fire and explosion, can be reduced.

Finally, the method for preparing a catalyst for preparing (meth)acrylic acid according to the present invention can provide an excellent catalyst for preparing (meth)acrylic acid, which has a high conversion rate of (meth)acrolein and a high selectivity for (meth)acrylic acid.

[Best Mode]

Hereinafter, the present invention will be described in detail such that a person skilled in the art to which the present invention pertains can easily carry out the present invention. However, the present invention may be implemented in various different forms, and is not limited to the configurations described herein.

When one part "includes" one constituent element in the present specification, unless otherwise specifically described, this does not mean that another constituent element is excluded, but means that another constituent element may be further included.

In the present specification, 'p to q' means 'p or more and q or less'.

In the present specification, the 'metal precursor' or 'precursor including an $M^k$ element" means a material which enables the preparation of a catalyst for preparing (meth)acrylic acid through a chemical reaction as a chemical including the metal or $M^k$ element, respectively. For example, when the metal is molybdenum, the above term is defined as a molybdenum precursor, and in this case, the molybdenum precursor means a material capable of preparing a catalyst for preparing (meth)acrylic acid through a chemical reaction as a chemical including molybdenum.

In the present specification, the notation of elements may be described based on the periodic table.

Further, in describing the present invention, detailed descriptions of related known techniques that may unnecessarily obscure the gist of the present invention will be omitted.

An exemplary embodiment of the present invention provides a method for preparing a catalyst for preparing (meth)acrylic acid represented by the following Chemical Formula 1, the method including: preparing a first solution including a molybdenum precursor and a vanadium precursor; preparing a second solution including a precursor including an $M^2$ element and a precursor including an $M^4$ element; preparing a catalyst suspension by mixing the first solution and the second solution; preparing a slurry by stirring and reacting the catalyst suspension; drying the slurry; pulverizing the dried slurry, and then adding a molding additive and molding the resulting mixture; and firing the molded catalyst according to the following Equation 1 while supplying a mixed gas including air and nitrogen ($N_2$).

[Chemical Formula 1]   $Mo_aV_bP_cM^1_dM^2_eM^3_fM^4_gM^5_hM^6_iO_j$

In Chemical Formula 1, Mo is molybdenum, V is vanadium, P is phosphorus, and O is oxygen,

$M^1$ is one or more elements selected from the group consisting of W, Sb, As, Sn, and Pb,

$M^2$ is one or more elements selected from the group consisting of Fe, Zn, Cr, Mn, and Cu,

$M^3$ is one or more elements selected from the group consisting of Se, Ga, Ti, Ge, Cd, Ta, and Ni,

$M^4$ is one or more elements selected from the group consisting of Al, Zr, Si, and Ce,

$M^5$ is one or more elements selected from the group consisting of Au, Pd, Pt, Ag, Ru, and Rh,

$M^6$ is one or more elements selected from the group consisting of Na, K, Li, Rb, Cs, Ca, Mg, Sr, and Ba,

a, b, c, d, e, f, g, h, i, and j represent the atomic proportion of each element, and

when a is 12, b is 0 to 2, c is 0 to 3, d is 0.001 to 15, e is 0.001 to 20, f is 0 to 20, g is 0 to 10, h is 0 to 1, i is 0.001 to 5, and j is a numerical value determined by the oxidation state of each component,

[Equation 1]

$$0.1\ \text{Lmin}^{-1}\text{g}^{-1} \le A/B \le 5\ \text{Lmin}^{-1}\text{g}^{-1}$$

in Equation 1,

A means the flow rate ($\text{Lmin}^{-1}$) of the mixed gas, and

B means the mass (g) of the molded catalyst.

In the present specification, the unit of the flow rate may be represented by L/min, and the A/B may be represented by a flow rate per gram of catalyst (L/min).

The method for preparing a catalyst for preparing (meth)acrylic acid according to the present invention can simplify and automate the process of preparing a catalyst by performing only one firing process, and can downsize the required equipment. Since the equipment may downsize as described above, the preparation process risk such as the leakage of chemicals and the risk of fire and explosion may be reduced. In addition, the method for preparing a catalyst for preparing (meth)acrylic acid according to the present invention may provide an excellent catalyst for preparing (meth) acrylic acid, which has a high conversion rate of (meth)acrolein and a high selectivity for (meth)acrylic acid.

Furthermore, Equation 1 may be $0.1\ \text{Lmin}^{-1}\text{g}^{-1} \le A/B \le 5\ \text{Lmin}^{-1}\text{g}^{-1}$, preferably $0.2\ \text{Lmin}^{-1}\text{g}^{-1} \le A/B \le 4.5\ \text{Lmin}^{-1}\text{g}^{-1}$. When Equation 1 is satisfied, a catalyst for preparing (meth)acrylic acid may be prepared more efficiently.

In the present invention, when the catalyst suspension is prepared, an acid such as nitric acid ($HNO_3$) may be added as a co-precipitant, a pore-forming agent, and/or a pH adjuster.

According to an exemplary embodiment of the present invention, Chemical Formula 1 may be represented by the following Chemical Formula 2. Specifically, the following Chemical Formula 2 may represent the composition ratio of elements except for oxygen.

[Chemical Formula 2]    $Mo_{12}V_{0.5}Cs_{1.5}Cu_{0.2}P_{1.3}Sb_{0.2}$

In an exemplary embodiment of the present invention, the preparing of the first solution including the molybdenum precursor and the vanadium precursor may include dissolving the molybdenum precursor and the vanadium precursor in a first solvent. In this case, it is possible to further include heating the first solvent.

In an exemplary embodiment of the present invention, the molybdenum precursor may be included in an amount of 10 parts by weight to 35 parts by weight, preferably 15 parts by weight to 25 parts by weight, based on 100 parts by weight of the first solution.

In an exemplary embodiment of the present invention, the vanadium precursor may be included in an amount of 0.5 parts by weight to 3 parts by weight, preferably 0.5 parts by weight to 2 parts by weight, based on 100 parts by weight of the first solution.

In an exemplary embodiment of the present invention, the first solvent may be water ($H_2O$).

In an exemplary embodiment of the present invention, the molybdenum precursor is preferably a salt soluble in water, nitric acid, and the like, and may be more preferably an ammonium salt, ammonium paramolybdate (($NH_4)_6Mo_7O_{24}$), or an oxide, molybdenum trioxide ($MoO_3$) but is not limited thereto. The molybdenum precursor may be a hydrate of the salt.

In an exemplary embodiment of the present invention, the vanadium precursor is preferably a salt soluble in water, nitric acid, and the like, and may be more preferably an ammonium salt, ammonium metavanadate ($NH_4VO_3$), or an oxide, vanadium pentoxide ($V_2O_5$), but is not limited thereto. The vanadium precursor may be a hydrate of the salt.

In an exemplary embodiment of the present invention, the preparing of the second solution including the precursor including the $M^2$ element and the precursor including the $M^4$ element may include dissolving the precursor including the $M^2$ element and the precursor including the $M^4$ element in a second solvent. In this case, it is possible to further include heating the second solvent.

In an exemplary embodiment of the present invention, the precursor including the $M^2$ element may be included in an amount of 0.5 parts by weight to 5 parts by weight, preferably 1 part by weight to 3.5 parts by weight, based on 100 parts by weight of the second solution.

In an exemplary embodiment of the present invention, the precursor including the $M^4$ element may be included in an amount of 5 parts by weight to 40 parts by weight, preferably 10 parts by weight to 25 parts by weight, based on 100 parts by weight of the second solution.

In an exemplary embodiment of the present invention, the second solvent may be one or more selected from the group consisting of water, phosphoric acid, and nitric acid, or a mixture thereof.

In an exemplary embodiment of the present invention, the precursor including the $M^2$ element may be a copper precursor. That is, $M^2$ may be Cu.

In an exemplary embodiment of the present invention, the copper precursor is preferably a salt soluble in water, nitric acid, phosphoric acid, and the like, and may be more preferably a nitrate, copper nitrate ($Cu(NO_3)_2$) or a carbonate, copper carbonate dihydroxide ($Cu_2(OH)_2CO_3$), but is not limited thereto. The copper precursor may be a hydrate of the salt.

In an exemplary embodiment of the present invention, the precursor including the $M^6$ element may be a cesium precursor. That is, $M^6$ may be Cs.

In an exemplary embodiment of the present invention, the cesium precursor is preferably a salt soluble in water, nitric acid, phosphoric acid, and the like, and may be more preferably a nitrate, cesium nitrate ($CsNO_3$), or a carbonate, cesium carbonate ($Cs_2CO_3$), but is not limited thereto. The cesium precursor may be a hydrate of the salt.

In an exemplary embodiment of the present invention, the preparing of the catalyst suspension by mixing the first solution and the second solution may include: cooling the first solution; and adding the second solution to the cooled first solution. The cooling of the first solution is to lower the temperature that increased during the process of preparing the first solution while heating the first solvent, and in this case, the temperature of the first solution may be cooled to about 60°C.

In an exemplary embodiment of the present invention, the preparing of the slurry by stirring and reacting the catalyst suspension may include: primarily stirring the catalyst suspension; adding an antimony precursor to the first stirred catalyst suspension; and secondarily stirring the catalyst suspension to which the antimony precursor is added.

In an exemplary embodiment of the present invention, the antimony precursor may be antimony trioxide ($Sb_2O_3$), more specifically a hydrate of antimony trioxide, but is not limited thereto.

In an exemplary embodiment of the present invention, the primarily stirring of the catalyst suspension may be performed for 15 minutes or more, 240 minutes or less, or 30 minutes or more and 120 minutes or less, but the duration is not limited thereto.

In an exemplary embodiment of the present invention, the secondarily stirring of the catalyst suspension may be performed for 1 hour or more, 48 hours or less, or preferably 5 hours or more and 24 hours or less.

That is, the time taken for the secondarily stirring of the catalyst suspension is longer than the time taken for the primarily stirring of the catalyst suspension.

In an exemplary embodiment of the present invention, the drying of the slurry may be performed at 100°C or higher, 300°C or less, preferably 100°C or higher, and 250°C or less. Further, the drying may be performed by any method in the related art, which is used in the art, for example, evaporative drying, atomization (spray) drying, drum drying, flash drying, and the like.

In an exemplary embodiment of the present invention, the molding additive may be those including an inorganic fiber and a pore-forming agent. As the pore-forming agent, ammonium nitrate may be used, but the pore-forming agent is not limited thereto.

In an exemplary embodiment of the present invention, the pulverizing of the dried slurry, and then the adding of the molding additive and the molding of the resulting mixture may include: preparing a mixture in which a molding additive, water, and alcohol are mixed after pulverizing the dried slurry; and molding the mixture. In this case, the molding of the mixture may be performed using a continuous extruder, but is not limited thereto.

In an exemplary embodiment of the present invention, the nitrogen in the mixed gas is present in gaseous form. Thus, the nitrogen in the mixed gas is described as $N_2$, which is in molecular form.

In an exemplary embodiment of the present invention, the volume of nitrogen in the mixed gas may be 1.5-fold to 30-fold, preferably 1.5-fold to 25-fold of the volume of air. When the volume proportion is satisfied, a catalyst for preparing (meth)acrylic acid may be prepared more efficiently.

In an exemplary embodiment of the present invention, the firing of the molded catalyst while supplying the mixed gas including air and nitrogen may be performed at a temperature of 300°C to 600°C, preferably 350°C to 550°C.

In an exemplary embodiment of the present invention, the mixed gas may further include water, and the water in the mixed gas may be included in an amount of 0.5 wt% to 15 wt%, preferably 1 wt% to 12 wt%, based on the total weight of the mixed gas. The content of the water means the content of water measured when the mixed gas is in a constant temperature bath at 5°C to 50°C.

An exemplary embodiment of the present invention provides a catalyst for preparing (meth)acrylic acid, which is prepared by the method for preparing (meth)acrylic acid of the present invention, in which the catalyst satisfies Chemical Formula 1. The catalyst for preparing (meth)acrylic acid, which is prepared by the method for preparing a catalyst for preparing (meth)acrylic acid according to the present invention, has a high conversion rate of (meth)acrolein, high selectivity for (meth)acrylic acid, and excellent performance.

An exemplary embodiment of the present invention provides a method for preparing (meth)acrylic acid, which prepares (meth)acrylic acid using the catalyst for preparing (meth)acrylic acid. Specifically, the method for preparing (meth)acrylic acid may prepare (meth)acrylic acid by filling a fixed-bed reactor with the catalyst for preparing (meth)acrylic acid, and performing the (meth)acrolein oxidation reaction while supplying (meth)acrolein.

In an exemplary embodiment of the present invention, the (meth)acrolein may be supplied at a space velocity of 5 $hr^{-1}$ to 100 $hr^{-1}$.

In an exemplary embodiment of the present invention, the (meth)acrolein oxidation reaction may be performed at a temperature of 300°C or higher.

The fixed-bed reactor applied to the method for preparing (meth)acrylic acid according to the present invention includes: a first catalyst layer filled with a front catalyst for preparing (meth)acrolein using propylene, isobutylene, or a mixture thereof as a raw material; a second catalyst layer filled with a rear catalyst for preparing (meth)acrylic acid using the (meth)acrolein as a raw material; and an inert material layer including an inert material between the first catalyst layer and the second catalyst layer.

Preferably, a known fixed-based reactor may be used as the fixed-bed reactor. Further, preferably, the fixed-bed phase reactor may be a fixed-bed multitubular reactor including a plurality of reaction tubes each including the first catalyst layer, the second catalyst layer, and the inert material layer.

Examples of the fixed-bed reactor include a fixed-bed reactor composed of one reactor equipped with a first-stage reaction zone (or front reaction zone) filled with the front catalyst and a second-stage reaction zone (or rear reaction zone) filled with the rear catalyst. In this case, the first-stage reaction zone corresponds to the first catalyst layer, the second stage reaction zone corresponds to the second catalyst layer, and the inert material layer is provided between the first-stage reaction zone and the second-stage reaction zone. That is, at least three layers composed of the first catalyst layer, the inert material layer, and the second catalyst layer are provided in one reaction tube.

As the fixed-bed reactor, a fixed-bed reactor composed of two reactors: a front reactor equipped with a reaction tube filled with the front catalyst and a rear reactor equipped with a reaction tube filled with the rear catalyst may also be used. In this case, the portion of the front reactor filled with the front catalyst corresponds to the first catalyst layer, and the portion of the rear reactor filled with the rear catalyst corresponds to the second catalyst layer. The inert material layer may be installed on the outlet side (the side from which gas is discharged in the reactor) rather than the first catalyst layer in the front reactor, or on the inlet side (the side into which the gas is introduced in the reactor) rather than the second catalyst layer in the rear reactor, or may be installed in a tube connecting the front reactor and the rear reactor.

As the reaction tube provided in the fixed-bed reactor, a typical reaction tube having a circular cross-sectional shape may be used. The inner diameter and length of the reaction tube may be determined within a range that allows the reaction tube to be filled with a catalyst and an inert material, and to exhibit an appropriate reaction efficiency. Preferably, the reaction tube may have an inner diameter of 15 mm to 100 mm and a length of 1 m to 10 m.

The front catalyst may convert propylene, isobutylene, or a mixture thereof into (meth)acrolein by the contact gas phase oxidation of propylene, isobutylene, or a mixture thereof, and the catalyst for preparing (meth)acrylic acid according to the present invention may be used as the front catalyst.

The rear catalyst may convert (meth)acrolein obtained from the first catalyst layer into (meth)acrylic acid by the contact gas phase oxidation of the (meth)acrolein, and the catalyst for preparing (meth)acrylic acid according to the present invention may be used.

In an exemplary embodiment of the present invention, the catalyst for preparing (meth)acrylic acid according to the present invention may be used as the front catalyst, and a known oxide catalyst may be used as the rear catalyst.

In an exemplary embodiment of the present invention, a known oxide catalyst may be used as the front catalyst, and the catalyst for preparing (meth)acrylic acid according to the present invention may be used as the rear catalyst.

In an exemplary embodiment of the present invention, the catalyst for preparing (meth)acrylic acid according to the present invention may be used as the front catalyst and the rear catalyst, respectively.

The front catalyst and the rear catalyst may each include an inert support along with the active component.

The composition of the material for the inert carrier is not particularly limited as long as it does not inhibit the activity of the catalyst. For example, the inert carrier may be one or more compounds selected from the group consisting of $SiO_2$, $Al_2O_3$, $MgO$, $MgCl_2$, $CaCl_2$, $ZrO_2$, $TiO_2$, $B_2O_3$, $CaO$, $ZnO$, $BaO$, $ThO_2$, $SiO_2$-$Al_2O_3$, $SiO_2$-$MgO$, $SiO_2$-$TiO_2$, $SiO_2$-$V_2O_5$, $SiO_2$-$Cr_2O_3$, $SiO_2$-$TiO_2$-$MgO$, and zeolite.

The front catalyst and the rear catalyst may each be prepared by a typical method in the art to which the present invention pertains.

For example, the catalysts may be prepared using an extrusion molding method, a tablet molding method, and the like, in which catalyst constituent components including the active component are molded into a predetermined shape.

In addition, the catalyst may be prepared by a supporting method in which the active component is supported on any inert carrier.

The shape of the catalysts is not particularly limited, and may be spherical, cylindrical, ring-shaped, or amorphous.

The first catalyst layer is filled with the front catalyst, and the second catalyst layer is filled with the rear catalyst.

As the front catalyst and the rear catalyst, a single type of catalyst may be each used, or multiple types of catalysts with different activities or configurations may be used.

Meanwhile, the inert material layer is filled between the first catalyst layer and the second catalyst layer. The inert material may be a material that exhibits substantially no activity against a (meth)acrolein-containing reaction gas and has heat resistance at above a temperature at which the reaction is performed. Preferably, the material for the inert material may be stainless steel.

In an exemplary embodiment of the present application, as the method for preparing (meth)acrylic acid, it is possible to apply a typically used method for preparing (meth)acrylic acid, except for using the catalyst for preparing (meth) acrylic acid of the present application.

[Mode for Invention]

Hereinafter, the present application will be described in detail with reference to Examples for specifically describing the present application. However, the Examples according to the present application may be modified in various forms, and it is not interpreted that the scope of the present application is limited to the Examples described in detail below. The Examples of the present application are provided for more completely explaining the present application to the person with ordinary skill in the art.

**<Example 1>**

A first solution was prepared by putting 1000 ml of distilled water into a 5 L reactor equipped with a stirrer and dissolving 300 g of $(NH_4)_6Mo_7O_{24} \cdot 4H_2O$ and 10 g of $NH_4VO_3 \cdot 4H_2O$.

Apart from this, a second solution was prepared by adding 50 g of $CsNO_3$, 8 g of $Cu(NO_3)_2$, 20 g of $H_3PO_4$, and 50 g of $HNO_3$ to 100 ml of high-temperature distilled water and mixing the resulting mixture. After the first solution was naturally cooled, a catalyst suspension was prepared by mixing the cooled first solution and the second solution. After the catalyst suspension was stirred for 15 minutes, a slurry was prepared by adding 3 g of $Sb_2O_3$ to the catalyst suspension and further stirring the resulting mixture at room temperature (20°C to 25°C) for 24 hours.

Thereafter, the slurry was added dropwise to a solution supply container of a spray dryer.

Subsequently, a powder with a size of 200 μm or less was obtained by drying the slurry added dropwise to the solution supply container using the spray dryer.

Thereafter, a catalyst was prepared by mixing the powder obtained through the spray dryer, an inorganic fiber as a molding additive, ammonium nitrate as a pore-forming agent, and a mixture of water and alcohol so as to satisfy 59 wt%, 20 wt%, 10 wt%, and 10 wt%, respectively, using a continuous mixer.

A molded catalyst was prepared by extrusion molding the mixed catalyst prepared by mixing the mixture so as to satisfy the above ratio through a continuous extruder.

Finally, a catalyst for preparing (meth)acrylic acid of Example 1 was prepared by heat-treating the molded catalyst at 400°C for 16 hours in a tubular firing furnace while supplying a firing gas at a total flow rate of 1 L/min per 1 (g) of catalyst. In this case, the firing gas included air and $N_2$, and the volume ratio of air and $N_2$ in the firing gas is as shown in the following Table 1.

**<Examples 2 and 3>**

The catalysts for preparing (meth)acrylic acid of Examples 2 and 3 were each prepared in the same manner as in Example 1, except that in Example 1, a firing gas satisfying the volume ratio of air and $N_2$ described in the following Table 1 was used.

**<Examples 4 and 5>**

The catalysts for preparing (meth)acrylic acid of Examples 4 and 5 were each prepared in the same manner as in Example 1, except that in Example 1, a firing gas satisfying the volume ratio of air and $N_2$ described in the following Table 1 and including water in the content described in the following Table 1 was used, and the flow rate was each changed as described in the following Table 1.

**<Comparative Example 1>**

A catalyst molded in the same manner as in Example 1 was prepared. Thereafter, the catalyst for preparing (meth)acrylic acid of Comparative Example 1 was prepared in the same manner as in Example 1, except that a firing gas including only $N_2$ without including air as shown in the following Table 1 was used.

**<Comparative Example 2>**

A catalyst molded in the same manner as in Example 1 was prepared. Thereafter, primary firing was performed by performing heat treatment at 400°C for 16 hours while supplying a firing gas including only $N_2$ without including air at a flow rate of 1 L/min per gram of catalyst. After the primary firing, the catalyst for preparing (meth)acrylic acid of Comparative Example 2 was prepared by performing secondary firing during a heat treatment at 400°C for 12 hours while supplying a firing gas including only air without including $N_2$ at a flow rate of 1 L/min.

In this case, the volume ratios of $N_2$ used in the primary firing and air used in the secondary firing were as shown in the following Table 1.

**<Comparative Examples 3 to 5>**

The catalysts for preparing (meth)acrylic acid of Comparative Examples 3 to 5 were each prepared in the same manner as in Comparative Example 1, except that in Comparative Example 1, firing gases including the volume ratio of air or $N_2$ and the content of water described in the following Table 1 were used. That is, in Comparative Examples 3 and 4, the firing gas did not include $N_2$, and in Comparative Example 5, the firing gas did not include air.

**<Comparative Example 6>**

A catalyst was prepared as follows.

A first solution was prepared by putting 4000 ml of distilled water into a 5 L reactor equipped with a stirrer and dissolving 300 g of $MoO_3$, 26 g of $H_3PO_4$, and 530 g of $V_2O_5$.

Apart from this, a second solution was prepared by adding 35 g of $CsCO_3$ to 50 ml of high-temperature distilled water and mixing the resulting mixture. After the first solution was naturally cooled, a catalyst suspension was prepared by mixing the cooled first solution and the second solution. After the catalyst suspension was stirred for 30 minutes, 4 g of $Cu_2(OH)_2CO_3$ and 5 g of $Sb_2O_3$ were added to the catalyst suspension, the resulting mixture was stirred at room temperature (20°C to 25°C) for 1 hour, and then 30 g of $NH_4NO_3$ was added thereto and the resulting mixture was additionally stirred for 30 minutes to prepare a slurry.

Thereafter, the slurry was added dropwise to a solution supply container of a spray dryer.

Subsequently, a powder of 200 $\mu$m or less was obtained by drying the slurry added dropwise to the solution supply container using the spray dryer.

Thereafter, a catalyst was prepared by mixing the powder obtained through the spray dryer, an inorganic fiber as a molding additive, ammonium nitrate as a pore-forming agent, and a mixture of water and alcohol so as to satisfy 62 wt%, 10 wt%, 10 wt%, and 17wt%, respectively, using a continuous mixer.

A molded catalyst was prepared by extrusion molding the mixed catalyst prepared by mixing the mixture so as to satisfy the above ratio through a continuous extruder.

Finally, the catalyst for preparing (meth)acrylic acid of Comparative Example 6 was prepared by heat-treating the molded catalyst in a tubular firing furnace at 400°C for 16 hours while supplying a firing gas at a flow rate of 0.08 L/min per gram of catalyst (that is, less than 0.1 $Lmin^{-1}g^{-1}$ and not satisfying Equation 1). In this case, the firing gas included air and $N_2$, and the volume ratio of air and $N_2$ in the firing gas is as shown in the following Table 1.

**<Comparative Example 7>**

A catalyst molded in the same manner as in Example 1 was prepared. Thereafter, the catalyst for preparing (meth)acrylic acid of Comparative Example 7 was prepared by performing primary firing, in which heat treatment is performed at 400°C for 16 hours while supplying a firing gas including only N2 without including air at a flow rate of 8 L/min per gram of catalyst (that is, more than 5 $Lmin^{-1}g^{-1}$ and not satisfying Equation 1) as shown in the following Table 1.

**<Experimental Examples: Catalyst activity test>**

A pilot-scale shell-and-tube type fixed bed reactor was prepared, which was composed of a steel reaction tube with an inner diameter of 1 inch and a fixed bed packed section length of 3000 mm, and a shell (diameter 100 mm) that covers the reaction tube and allows a heat medium to flow. The reactor included a front catalyst layer or a rear catalyst layer, the same catalyst was used in the front catalyst layer, and the rear catalyst layer was each filled with the catalysts of Examples 1 to 5 and Comparative Examples 1 to 7. Thereafter, the raw material mixed gas (7.5 vol% raw gas, 14 vol% oxygen, 18 vol% water vapor, and 60.5 vol% inert nitrogen gas) was flowed at a space velocity of 80 hr$^{-1}$ to perform the reaction at a reaction temperature of 310°C. The conversion rate and selectivity were calculated by comparing the number of moles of each produced material using a gas chromatography analyzer for the gas that passed through the reactor.

As the raw material gas mixture (raw material gas), one of propylene, isobutylene, t-butyl alcohol, and methyl-t-butyl ether, or a mixture thereof may be used, but in this experimental example, an experiment was conducted when the raw material gas included propylene and isobutylene.

- Gas chromatography operating conditions

Instrument: Agilent 7890B Gas Chromatography system

Column: Molecular sieve 5A 2.1 mm (L.D)*2.0 M 80/100 mesh

Plot Q 2.1 mm (I.D)*2.0 M 80/100 mesh

WAX-DA 0.53 mm (I.D)*30 M*1 μm

Solvent: 1,4-dioxane

Column temperature: measured while increasing the temperature from 50°C to 200°C

Moisture content: measured at 120°C for 10 minutes using an MX-50 moisture content apparatus

Based on the analyzed results, the conversion rate and selectivity yield were calculated and are shown in the following Table 2.

Conversion rate (%) (MACR Conv.) = [(the number of moles of reacted (meth) acrolein) / (the number of moles of supplied (meth)acrolein)]*100(%) Selectivity (%) (MAA SEL.)= [(the number of moles of produced (meth)acrylic acid)/(the number of moles of reacted acrolein)]*100(%) Yield (%) (MAA Yield) = [(the number of moles of produced (meth)acrylic acid)/(the number of moles of supplied (meth)acrolein)] *100(%)

[Table 1]

| No. | Firing step | | | | Firing time [h] | | Catalyst Mass (g) | Whether Chemical Formula 1 is satisfied |
|---|---|---|---|---|---|---|---|---|
| | Primary firing | Secondary firing | Air: $N_2$ (volume ratio) | per gram of catalyst Flow rate (L/min) (Whether Equation 1 is satisfied) | Primary firing | Secondary firing | | |
| Example 1 | Air and $N_2$ Mixed | - | 2.5 : 7.5 | 1 (Satisfactory) | 16 | - | 200 to 1,000 g | Satisfactory |
| Example 2 | Air and $N_2$ mixed | - | 1 : 9 | 1 (Satisfactory) | 16 | - | 200 to 1,000 g | Satisfactory |
| Example 3 | Air and $N_2$ Mixed | - | 0.5 : 9.5 | 1 (Satisfactory) | 16 | - | 200 to 1,000 g | Satisfactory |
| Example 4 | Air and $N_2$ Mixed (+water) | - | 0.5 : 9.5 (water 3 wt%) | 0.1 (Satisfactory) | 16 | - | 200 to 1,000 g | Satisfactory |
| Example 5 | Air and $N_2$ mixed (+water) | - | 0.5 : 9.5 (water 7 wt%) | 5 (Satisfactory) | 16 | - | 200 to 1,000 g | Satisfactory |
| Comparative Example 1 | $N_2$ | - | 0 : 10 | 1 (Satisfactory) | 16 | - | 200 to 1,000 g | Satisfactory |

(continued)

| No. | Firing step | | | | Firing time [h] | | Catalyst Mass (g) | Whether Chemical Formula 1 is satisfied |
|---|---|---|---|---|---|---|---|---|
| | Primary firing | Secondary firing | Air: N$_2$ (volume ratio) | per gram of catalyst Flow rate (L/min) (Whether Equation 1 is satisfied) | Primary firing | Secondary firing | | |
| Comparative Example 2 | N$_2$ | Air | 5 : 5 | 1 (Satisfactory) | 16 | 12 | 200 to 1,000 g | Satisfactory |
| Comparative Example 3 | Air (+water) | - | 10 : 0 (water 3 wt%) | 1 (Satisfactory) | 16 | - | 200 to 1,000 g | Satisfactory |
| Comparative Example 4 | Air (+water) | - | 10 : 0 (water 7 wt%) | 1 (Satisfactory) | 16 | - | 200 to 1,000 g | Satisfactory |
| Comparative Example 5 | N$_2$ (+water) | - | 0 : 10 (water 3 wt%) | 1 (Satisfactory) | 16 | - | 200 to 1,000 g | Satisfactory |
| Comparative Example 6 | Air and N$_2$ Mixed | - | 5 : 5 | 0.08 (Unsatisfactory) | 16 | - | 200 to 1,000 g | Satisfactory |
| Comparative Example 7 | N$_2$ | - | 0 : 10 | 8 (Unsatisfac-tory) | 16 | - | 200 to 1,000 g | Satisfactory |

In Table 1, the materials described in the firing step mean the materials which the gases used in each firing step include, and + water means that water is included in the gas. Further, when the firing step was performed only once, the relevant content was described for the primary firing. Accordingly, in Table 1 above, the firing time was described for each firing step, and when the secondary firing step was not performed, the time related to the secondary firing was described as '-'. That is, the total firing time can be said to be the time taken for primary firing and secondary firing.

[Table 2]

| Catalyst used | MACR Conv. [%] | MAA SEL. [%] | MAA Yield. [%] |
|---|---|---|---|
| Example 1 | 92.9 | 74.2 | 62.25 |
| Example 2 | 92.2 | 76.9 | 64.02 |
| Example 3 | 91.5 | 78.9 | 65.19 |
| Example 4 | 94.1 | 78.9 | 67.04 |
| Example 5 | 81.6 | 86.2 | 63.52 |
| Comparative Example 1 | 93.5 | 71.9 | 60.71 |
| Comparative Example 2 | 93.7 | 69.6 | 58.89 |
| Comparative Example 3 | 54.9 | 79.5 | 39.41 |
| Comparative Example 4 | 29.6 | 78.5 | 20.98 |
| Comparative Example 5 | 63.8 | 88.7 | 51.10 |
| Comparative Example 6 | 63.7 | 82.42 | 47.41 |
| Comparative Example 7 | 90.13 | 66.17 | 53.85 |

The catalysts used in Table 2 above mean that the rear catalyst layer is filled with the catalysts prepared in Examples 1 to 5 and Comparative Examples 1 to 7 as described above.

Referring to the results of Tables 1 and 2, in the method for preparing a catalyst for preparing (meth)acrylic acid of Examples 1 to 5, in which a mixed gas of air and $N_2$ is used simultaneously in one step of firing, it is possible to confirm the effect of improving the performance aspect of the catalyst or shortening the time for preparing the catalyst than in Comparative Examples 1 to 7, in which air or $N_2$ is used alone instead of the mixed gas of air and $N_2$ or the two-step firing is performed.

Specifically, when the catalysts of Comparative Examples 1 and 7, which were fired using only $N_2$ gas, were used, it could be confirmed that under the conditions in which the catalyst preparation time is the same, the yield of (meth)acrylic acid and the selectivity of the yield of (meth)acrylic acid were lower than when the air and $N_2$ firing gas (mixed gas) were used as in Examples 1 to 5. Moreover, when determined together with the results of Comparative Examples 2 and 5, it could be confirmed that using $N_2$ gas alone may have an adverse effect on the yield of (meth)acrylic acid and the selectivity of the yield of (meth)acrylic acid.

In contrast, when the catalysts of Comparative Examples 3 and 4, which were fired using only air gas, were used, it could be confirmed that under the conditions in which the catalyst preparation time is the same, the yield of (meth)acrylic acid and the selectivity of the yield of (meth)acrylic acid were remarkably lower than when the catalysts of Examples 1 to 5 were used. It could be confirmed that the performance is not good enough to be used as a catalyst for preparing (meth)acrylic acid when $N_2$ gas is not used.

Further, from the results of Comparative Example 2, it could be confirmed that the order in which the types of gas used in the primary firing and the gas used in the secondary firing are used may also affect catalyst performance. In contrast, since the method for preparing a catalyst for preparing (meth)acrylic acid according to the present invention uses a mixed gas of air and $N_2$, there is no need to consider the order in which different types of firing gases are introduced. This means that the method for preparing a catalyst for preparing (meth)acrylic acid according to the present invention is more convenient in terms of process.

Furthermore, when compared to Examples 1 to 5 in which the catalyst satisfies Chemical Formula 1 and the flow rate per gram of catalyst satisfies Equation 1, it could be confirmed that even though catalysts satisfying Chemical Formula 1 are used as in Comparative Examples 6 and 7, the performance (conversion rate, selectivity, and/or yield) of the catalyst was poor because the flow rate per gram of catalyst did not match Equation 1 of the present invention.

Additionally, referring to Examples 4 and 5, it could be confirmed that when the air and $N_2$ mixed gas (firing gas) includes water (moisture), it is possible to further increase the yield of (meth)acrylic acid or the selectivity of the yield of (meth)acrylic acid.

That is, as described above, the method for preparing a catalyst for preparing (meth)acrylic acid according to the present invention may prepare a catalyst with excellent performance within a short period of time, meaning that it is easier to increase productivity and simplify equipment in the process of preparing a catalyst.

**Claims**

1. A method for preparing a catalyst for preparing (meth)acrylic acid represented by the following Chemical Formula 1, the method comprising:

    preparing a first solution comprising a molybdenum precursor and a vanadium precursor;
    preparing a second solution comprising a precursor comprising an $M^2$ element and a precursor comprising an $M^4$ element;
    preparing a catalyst suspension by mixing the first solution and the second solution;
    preparing a slurry by stirring and reacting the catalyst suspension;
    drying the slurry;
    pulverizing the dried slurry, and then adding a molding additive and molding the resulting mixture; and
    firing the molded catalyst according to the following Equation 1 while supplying a mixed gas comprising air and nitrogen ($N_2$):

    [Chemical Formula 1]        $Mo_aV_bP_cM^1_dM^2_eM^3_fM^4_gM^5_hM^6_iO_j$

    wherein, in Chemical Formula 1, Mo is molybdenum, V is vanadium, P is phosphorus, and O is oxygen,
    $M^1$ is one or more elements selected from the group consisting of W, Sb, As, Sn, and Pb,
    $M^2$ is one or more elements selected from the group consisting of Fe, Zn, Cr, Mn, and Cu,
    $M^3$ is one or more elements selected from the group consisting of Se, Ga, Ti, Ge, Cd, Ta, and Ni,
    $M^4$ is one or more elements selected from the group consisting of Al, Zr, Si, and Ce,
    $M^5$ is one or more elements selected from the group consisting of Au, Pd, Pt, Ag, Ru, and Rh,
    $M^6$ is one or more elements selected from the group consisting of Na, K, Li, Rb, Cs, Ca, Mg, Sr, and Ba,
    a, b, c, d, e, f, g, h, i, and j represent the atomic proportion of each element, and

when a is 12, b is 0 to 2, c is 0 to 3, d is 0.001 to 15, e is 0.001 to 20, f is 0 to 20, g is 0 to 10, h is 0 to 1, i is 0.001 to 5, and j is a numerical value determined by the oxidation state of each component,

[Equation 1]

$$0.1 \ Lmin^{-1}g^{-1} \leq A/B \leq 5 \ Lmin^{-1}g^{-1}$$

in Equation 1,
A means the flow rate ($Lmin^{-1}$) of the mixed gas, and
B means the mass (g) of the molded catalyst.

2. The method of claim 1, wherein the volume of nitrogen in the mixed gas is 1.5-fold to 30-fold of the volume of air.

3. The method of claim 1, wherein the firing of the molded catalyst while supplying the mixed gas comprising air and nitrogen is performed at a temperature of 300°C to 600°C.

4. The method of claim 1, wherein the mixed gas further comprises water.

5. The method of claim 4, wherein the water in the mixed gas is comprised in an amount of 0.5 wt% to 15 wt% based on the total weight of the mixed gas.

6. The method of claim 1, wherein the molding additive comprises an inorganic fiber and a pore-forming agent.

7. The method of claim 1, wherein the preparing of the slurry by stirring and reacting the catalyst suspension comprises:

primarily stirring the catalyst suspension;
adding an antimony precursor to the first stirred catalyst suspension; and
secondarily stirring the catalyst suspension to which the antimony precursor is added.

8. The method of claim 1, wherein the pulverizing of the dried slurry, and then the adding of the molding additive and the molding of the resulting mixture comprises:

preparing a mixture in which a molding additive, water, and alcohol are mixed after pulverizing the dried slurry; and
molding the mixture.

9. A catalyst for preparing (meth)acrylic acid prepared by the method of any one of claims 1 to 8,

wherein the catalyst satisfies the following Chemical Formula 1:

[Chemical Formula 1]     $Mo_aV_bP_cM^1_dM^2_eM^3_fM^4_gM^5_hM^6_iO_j$

in Chemical Formula 1, Mo is molybdenum, V is vanadium, P is phosphorus, and O is oxygen,
$M^1$ is one or more elements selected from the group consisting of W, Sb, As, Sn, and Pb,
$M^2$ is one or more elements selected from the group consisting of Fe, Zn, Cr, Mn, and Cu,
$M^3$ is one or more elements selected from the group consisting of Se, Ga, Ti, Ge, Cd, Ta, and Ni,
$M^4$ is one or more elements selected from the group consisting of Al, Zr, Si, and Ce,
$M^5$ is one or more elements selected from the group consisting of Au, Pd, Pt, Ag, Ru, and Rh,
$M^6$ is one or more elements selected from the group consisting of Na, K, Li, Rb, Cs, Ca, Mg, Sr, and Ba,
a, b, c, d, e, f, g, h, i, and j represent the atomic proportion of each element, and
when a is 12, b is 0 to 2, c is 0 to 3, d is 0.001 to 15, e is 0.001 to 20, f is 0 to 20, g is 0 to 10, h is 0 to 1, i is 0.001 to 5, and j is a numerical value determined by the oxidation state of each component.

## INTERNATIONAL SEARCH REPORT

| International application No. |
| --- |
| **PCT/KR2024/000673** |

| | |
| --- | --- |
| **A.** | **CLASSIFICATION OF SUBJECT MATTER** |

**B01J 37/00**(2006.01)i; **B01J 37/08**(2006.01)i; **B01J 37/04**(2006.01)i; **B01J 27/199**(2006.01)i; **C07C 51/25**(2006.01)i; **C07C 57/04**(2006.01)i

According to International Patent Classification (IPC) or to both national classification and IPC

| | |
| --- | --- |
| **B.** | **FIELDS SEARCHED** |

Minimum documentation searched (classification system followed by classification symbols)

B01J 37/00(2006.01); B01J 23/26(2006.01); B01J 23/30(2006.01); B01J 23/644(2006.01); B01J 23/76(2006.01); B01J 23/887(2006.01); B01J 23/888(2006.01); B01J 27/18(2006.01); B01J 27/199(2006.01); B01J 38/02(2006.01); C07C 51/25(2006.01); C07C 57/055(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 촉매(catalyst), (메타)아크릴산((meth)acrylic acid), 몰리브덴 (molybdenum), 바나듐(vanadium), 전구체(precursor), 공기(air), 질소(nitrogen), 소성(calcination)

| | |
| --- | --- |
| **C.** | **DOCUMENTS CONSIDERED TO BE RELEVANT** |

| Category* | Citation of document, with indication, where appropriate, of the relevant passages | Relevant to claim No. |
| --- | --- | --- |
| Y | KR 10-2021-0039216 A (LG CHEM, LTD.) 09 April 2021 (2021-04-09) <br> See claims 1-4; and paragraphs [0125]-[0130]. | 1-9 |
| Y | KR 10-2000-0039469 A (LG CHEM, LTD.) 05 July 2000 (2000-07-05) <br> See paragraphs [0031]-[0035] and [0047]-[0049]; and table 1 (example 11). | 1-9 |
| Y | KR 10-2012-0106634 A (SUMITOMO CHEMICAL CO., LTD.) 26 September 2012 (2012-09-26) <br> See paragraphs [0044]-[0045]. | 4,5 |
| Y | JP 2018-153777 A (NIPPON SHOKUBAI CO., LTD.) 04 October 2018 (2018-10-04) <br> See paragraphs [0016] and [0023]. | 6,8 |
| A | KR 10-2021-0097620 A (SUMITOMO CHEMICAL CO., LTD.) 09 August 2021 (2021-08-09) <br> See entire document. | 1-9 |

| | | |
| --- | --- | --- |
| ✓ Further documents are listed in the continuation of Box C. | | ✓ See patent family annex. |

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
| --- | --- |
| **01 May 2024** | **01 May 2024** |

| Name and mailing address of the ISA/KR | Authorized officer |
| --- | --- |
| **Korean Intellectual Property Office** <br> **Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

International application No.

**PCT/KR2024/000673**

| | C. DOCUMENTS CONSIDERED TO BE RELEVANT | |
|---|---|---|
| Category* | Citation of document, with indication, where appropriate, of the relevant passages | Relevant to claim No. |
| A | KR 10-0896379 B1 (MITSUBISHI RAYON CO., LTD.) 08 May 2009 (2009-05-08)<br>See entire document. | 1-9 |

Form PCT/ISA/210 (second sheet) (July 2022)

## INTERNATIONAL SEARCH REPORT
### Information on patent family members

International application No.

**PCT/KR2024/000673**

| Patent document cited in search report | | | Publication date (day/month/year) | Patent family member(s) | | | Publication date (day/month/year) |
|---|---|---|---|---|---|---|---|
| KR | 10-2021-0039216 | A | 09 April 2021 | None | | | |
| KR | 10-2000-0039469 | A | 05 July 2000 | KR | 10-0378018 | B1 | 19 August 2003 |
| KR | 10-2012-0106634 | A | 26 September 2012 | JP | 2012-196608 | A | 18 October 2012 |
| JP | 2018-153777 | A | 04 October 2018 | None | | | |
| KR | 10-2021-0097620 | A | 09 August 2021 | CN | 113262806 | A | 17 August 2021 |
| | | | | CN | 113262806 | B | 26 March 2024 |
| | | | | JP | 2021-120333 | A | 19 August 2021 |
| | | | | JP | 7356923 | B2 | 05 October 2023 |
| KR | 10-0896379 | B1 | 08 May 2009 | CN | 100874842 | A | 06 December 2006 |
| | | | | CN | 100874842 | B | 22 June 2011 |
| | | | | JP | 2007-039760 | A1 | 22 February 2007 |
| | | | | JP | 4922614 | B2 | 25 April 2012 |
| | | | | KR | 10-2006-0076320 | A | 04 July 2006 |
| | | | | SG | 156608 | A1 | 26 November 2009 |
| | | | | US | 2007-0032679 | A1 | 08 February 2007 |
| | | | | US | 7662742 | B2 | 16 February 2010 |
| | | | | WO | 2005-039760 | A1 | 06 May 2005 |

Form PCT/ISA/210 (patent family annex) (July 2022)

**EP 4 653 090 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020230008209
- KR 1020240005265